(19) **Europäisches Patentamt** / **European Patent Office** / **Office européen des brevets**

(11) **EP 3 425 380 A1**

(12) # EUROPEAN PATENT APPLICATION

(43) Date of publication:
**09.01.2019 Bulletin 2019/02**

(51) Int Cl.:
*G01N 27/02* (2006.01)  *G01N 33/12* (2006.01)

(21) Application number: **18184665.0**

(22) Date of filing: **23.12.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **20.01.2014 ES 201430053**

(62) Document number(s) of the earlier application(s) in accordance with Art. 76 EPC:
**14844970.5 / 3 098 597**

(71) Applicant: **Lenz Instruments S.L.**
**08940 Cornellà de Llobregat (ES)**

(72) Inventors:
- **RODRÍGUEZ VENTURA, Juan Manuel**
  **08018 Barcelona (ES)**
- **ÁLVAREZ GARCÍA, Jacobo**
  **08012 Barcelona (ES)**
- **WILLIAMS HALLOWS, Mark Richard**
  **08173 Sant Cugat del Vallès (ES)**

(74) Representative: **Curell Suñol S.L.P.**
**Via Augusta 21**
**08006 Barcelona (ES)**

Remarks:
This application was filed on 20-07-2018 as a divisional application to the application mentioned under INID code 62.

(54) **METHOD FOR DETERMINING QUALITY PARAMETERS IN MEAT PRODUCTS AND CORRESPONDING INSTALLATION**

(57)    The present invention relates to a method for determining quality parameters in meat products and the corresponding installation. The method comprises the steps of providing at least one transmitter coil (2) and one receiver coil (4), said transmitter coil (2) generating a primary variable electromagnetic field, subjecting the meat product (50) to the primary field generating induced currents in the meat product (50) and detecting the resulting total electromagnetic field by means of said receiver coil (4). The method furthermore envisages the steps of obtaining the in-phase and/or quadrature components of the total electromagnetic field, and obtaining an estimate of the quality parameters of said meat product (50) based on the in-phase and/or quadrature components of the total field using pre-established models of correlation between the in-phase and/or quadrature components and a parameter of the group consisting of water-holding capacity, salt uptake in lean and/or intramuscular fat content.

FIG.1

**Description**

Field of the invention

The invention relates to a method for determining quality parameters in meat products comprising the steps of providing at least one transmitter coil and at least one receiver coil, generating a primary time-variable electromagnetic field by means of said at least one transmitter coil, subjecting said meat product to the effects of said primary electromagnetic field for generating currents induced in said meat product, and establishing a secondary electromagnetic field, and detecting the total electromagnetic field by means of said at least one receiver coil.

The invention also relates to an installation for determining quality parameters in meat products comprising at least one transmitter coil, at least one receiver coil, generating means for generating a primary time-variable electromagnetic field in said at least one transmitter coil, determination means for determining in said at least one receiver coil the in-phase and/or quadrature components of the total electromagnetic field.

Definitions

The term "meat products" in the present invention must be interpreted in a broad sense. In this sense, the concept includes all fresh, processed or semi-processed products of the meat industry, including meat of pork, veal, lamb, chicken, turkey and horse. For example, in the case of pork, the invention can be applied to cuts of meat that result from cutting (such as hams, shoulders, bellies, tenderloins or others), meat trimmings, minced meat or any other specialty in the charcuterie sector. In addition, the term "meat product" must be considered as the meat from any animal intended for human consumption, such as fish meat.

In the invention, the term "salt uptake in lean" relates to the amount of salt taken up in the loin of the meat product after being subjected to a dry salting process (as in the case of the Spanish cured ham) or in brine (such as applying a brine injection for cooked ham, for example).

Furthermore, in the invention the term "intramuscular fat" relates to the fat that has infiltrated lean tissue muscle fibers.

Additionally, in order to avoid any ambiguity, the following definitions are provided:

- Primary electromagnetic field: electromagnetic field generated in space by the at least one transmitter coil as a result of the circulation of a current having a time-variable amplitude in said transmitter coil.
- Secondary electromagnetic field: electromagnetic field generated in space by the currents induced in the meat product as a result of being subjected to the action of the primary electromagnetic field.
- Total electromagnetic field: electromagnetic field that results from the superposition of the primary electromagnetic field and secondary electromagnetic field and is detected based on the current induced in said at least one receiver coil.
- In-phase and quadrature components of the total electromagnetic field: components resulting from the vector decomposition of the total electromagnetic field, and having a 0° and 90° phase shift, respectively, relative to the primary electromagnetic field.

State of the art

In addition to the chemical composition of a meat product in terms of its total fat and loin content, there are a number of variables relating to its final quality. Among such variables, the following group of parameters related in a complex manner with both the physicochemical characteristics of the meat product and with its tissue and cell structure, must be pointed out: (a) water-holding capacity, also referred to as WHC, (b) salt uptake in lean, defined based on the amount of salt taken up in the product muscle tissue (loin) upon subjecting it to a salting process, and (c) the intramuscular fat content in the product.

In general, the relationship between these quality parameters and physicochemical and structural properties of the product is complex. In the case of water-holding capacity, this parameter is fundamentally conditioned by a series of processes, such as chemical decomposition of glycogen present in the muscle after slaughter, the formation of lactic acid, and evolution of muscle tissue pH. Associated with said changes, changes take place in the cell structure of the meat product on a cell membrane level. Such physicochemical processes and structural modifications ultimately determine the capacity of meat product to hold water within its structure.

Similarly, a meat product salting process also entails significant variations in its properties. At the physicochemical level, this process progresses through a dual diffusion process, whereby salt diffuses into the product and water diffuses towards the surface. Furthermore, as a result of the interaction of muscle tissue with salt ions and additives that are introduced, chemical and enzymatic reactions, as well as changes in the cell structure of the product, take place.

Finally, the intramuscular fat content of a meat product is directly related to the distribution of fat and muscle tissue in the product. Many times, as in the case of Iberian cured hams, the distribution of intramuscular fat is not homogenous and does not have a direct relationship with the total fat content of the product.

In the meat industry, determining the quality parameters of meat products (water-holding capacity, salt uptake in lean and intramuscular fat content) is extremely important for being able to adjust production processes and assure uniform product quality. Furthermore, this allows establishing production segmentation strategies, and in many cases, maximizing industrial output of the production process. Currently, these quality parameters can only be monitored by means of destructive and/or invasive methods. These methods have a number of limitations in terms of analysis time, automation for being implemented on the line, cost, maintenance, risk of cross contamination, and/or precision. Despite such limitations, the complex relationship between said quality parameters and product properties has hindered development of non-destructive inspection methods.

Among the quality parameters that are considered, water-holding capacity is particularly relevant because it has a considerable effect on both the organoleptic quality of the end product, and on the output of many industrial production processes. Raw material having insufficient water-holding capacity, such as pale, soft and exudative meats (PSE), or having excessive water-holding capacity, such as dark, firm and dry meats (DFD), cause defects in either fresh or prepared end products. These defects lower organoleptic quality of the product and reduce industrial output. The main defects are excessive weight loss, texture problems, food stability and safety problems, discolouration, inadequate salt uptake, defects when slicing the product, such as tears, cracks, holes, etc.

The water-holding capacity is estimated today by means of monitoring the progression of meat pH after slaughter, usually after 45 minutes ($pH_{45}$) and/or 24 hours ($pH_{24}$). Monitoring is done by means of needle shaped electrodes which are subjected to wear and eventual oxidation. Furthermore, this system has the added problem that the measurement is only representative of a limited volume of the meat product being analyzed.

Alternatively, in some cases the water-holding capacity can be estimated by means of measuring color in the lean area. The color can be objectively measured with a Minolta CR-400 colorimeter (or similar) or by means of a subjective visual inspection with well-trained operators. A limitation of the method of measuring by means of the colorimeter is the error introduced in the color measurement due to the presence of connective tissue and/or fat. On the other hand, this method alone does not allow determining a certain type of meats having a water-holding capacity problem, such as red, soft and exudative meats (RSE). This type of meat is present in a high percentage, around 35% of pork production.

In addition, and in relation to cured and salted product production processes, it is important to determine the amount of salt taken up by the processed meat for the purpose of assuring specific food quality and safety. Although there are a number of experimental methods for determining the content and distribution of salt in meat products, such as nuclear magnetic resonance imaging, most of them are complex and expensive to implement as quality control procedures in production lines. The instrument commonly used today in the meat industry is the salinometer, consisting of an invasive probe that allows evaluating conductivity of the cut. However, like in the case of measuring water-holding capacity, these measurements only provide information corresponding to a limited sample volume and are furthermore subject to errors associated with variability of the contact resistance between the electrode and the medium and with the geometry of the cut.

Another problem associated with measuring these quality parameters with the described probes consists of the difficulty in automating processes for determining these parameters.

In addition, the intramuscular fat content is an important quality parameter because it is closely related to the flavor, aroma and tenderness of the meat. A minimum amount of intramuscular fat (usually 2%) is necessary for obtaining optimal sensory quality of fresh meat. Usually, values comprised between 3 and 4% are more suitable for meat intended for cured products. In addition, in the production of cured meat (ham, tenderloin, etc.), intramuscular fat acts like a physical barrier to the release of water, thus preventing the cuts of meat from becoming excessively dehydrated. In addition, it also acts like a physical barrier against the entry of salt into the inner portions of the ham, which ultimately increases salting and curing times. Determining the intramuscular fat content in cuts of meat, in addition to enabling segmentation of the raw material and more optimally modifying production processes, allows meat processers to establish a fair payment criterion with the supplier of said raw material based on its quality.

Experimental methods for determining intramuscular fat are destructive, including chemical extraction or determination by means of near-infrared spectroscopy, also referred to as NIRS, among others.

Summary of the invention

It is an object of the invention to provide a method and an installation of the type indicated above, to determine quality parameters in meat products by non-invasive methods and furthermore, to offer information that is more representative of the product as a whole. Particularly, the main objective of the invention is to determine the water-holding capacity and/or salt uptake in lean and/or intramuscular fat content, in any type of meat products, whether they are fresh or semi-processed cuts or cured or salted products.

In reference to the installation, it is not discarded that said installation may allow determining quality parameters other than those of the method according to the invention.

This purpose is achieved by means of a method for determining quality parameters in meat products of the type indicated above, characterized in that it further comprises the steps of obtaining from said receiver coil the in-phase and quadrature components of the total electromagnetic field, and obtaining an estimate of said quality parameters of said meat product based on said in-phase and/or quadrature components of the total electromagnetic field, using pre-established models of correlation between said in-phase and/or quadrature components of the total electromagnetic field and at least one parameter of the group consisting of water-holding capacity, salt uptake in lean and/or intramuscular fat content, where said models of correlation have been obtained from prior experimental tests.

Equivalently to determining the in-phase and quadrature components, the method could include determining the modulus and phase of the signal induced in the receiver coil or, where appropriate, multiple receiver coils.

The method according to the invention has multiple advantages with respect to the invasive methods. First, the meat products remain intact, and furthermore the risk of cross contaminations between different products is eliminated because it does not require probes to be inserted into the products. In addition, the mentioned problem of the overall estimate based on a local measurement is also solved because the present method allows analyzing the entire product as a whole. Furthermore, since the meat product is not handled with the probe, the system allows gaining inspection speed, thereby allowing implementation in completely automated sorting lines.

The described method is a powerful method of characterization, which allows determining quality parameters in meat products, as experimentally confirmed. In general, the relationship between said quality parameters and the electromagnetic response of the meat product is complex. First, it must be taken into account that it is not possible to establish a simple relationship between the quality parameters of the meat product and its physicochemical and structural properties. On the other hand, variations in the electromagnetic response of the product as a result of physicochemical and structural changes cannot be established in advance in a trivial manner either.

The method described in the present invention is based on analyzing variables affecting quality parameters in meat products, and on observing that these variables modify to a greater or lesser extent the magnitude and spatial distribution of the flow of currents induced in the meat product subjected to the action of a primary time-variable electromagnetic field. The developed technique preferably comprises using various excitation frequencies, which allows characterizing the response of the meat product giving priority to a specific mechanism of conduction. In general, working frequencies in the range of kHz (kilohertz) or tens of kHz are suitable for characterizing mechanisms of ion conduction, as well as the distribution and morphology of the product tissues. Complementarily, the frequency range of 100 kHz to 100 MHz (megahertz) allows characterizing the cell structure of the meat product through the mechanism of dispersion associated with the cell membrane of the constituent tissues, commonly known as mechanism of $\beta$-dispersion. Therefore, the use of multiple frequencies allows indirectly evaluating the physicochemical and structural properties of the meat product based on its electromagnetic response. The developed method is therefore based on experimentally establishing correlation models between the electromagnetic response of the meat product, through the in-phase and quadrature components of the total electromagnetic field, and the previously described quality parameters. It should also be pointed out that decomposition of the total electromagnetic field into its in-phase and quadrature components relative to the primary electromagnetic field is particularly advantageous, because it allows separating the contributions to the secondary electromagnetic field associated with the real part (in-phase component) and the imaginary part (quadrature component) of the dielectric constant of the meat product.

The invention further includes a number of preferred features that are object of the dependent claims and the utility of which will be highlighted hereinafter in the detailed description of an embodiment of the invention.

In a preferred embodiment, the method further comprises the steps of obtaining the initial weight of said meat product, adding said initial weight to said models of correlation and obtaining an estimated value of the water-holding capacity and/or salt uptake in lean and/or intramuscular fat content of said meat product based on said models of correlation. The initial weight can be obtained in a simple manner because it is either known at the entry into the installation or the installation can be equipped with a scale or weighing system. As an advantage to highlight, the initial weight parameter allows considerably improving the in-line estimate of the WHC, salt uptake in lean and intramuscular fat content. Such improvement is fundamentally related to the fact that mutual inductance between the meat product and the transmitter and receiver coils increases with the sample volume. In addition to this factor, it must also be taken into account that the amplitude of the secondary electromagnetic field increases with product mass. Therefore, the introduction of linear and/or non-linear terms including the weight of the product allows establishing more precise models of correlation.

In a preferred embodiment, the method further comprises the steps of obtaining weight loss after salting as a difference between the initial weight of said meat product and the final weight after salting, adding said weight loss to said models of correlation and obtaining an estimated value of the salt uptake in lean of said meat product based on said models of correlation. Weight loss after the salting process provides a direct measurement of the amount of water that the product has lost as a consequence of the diffusion of water towards the surface. Including this variable in models of correlation allows solving the uncertainty associated with the fact that the dielectric properties of the meat product depend on both salt uptake and on the drip loss in the diffusion process. Accordingly, the prediction error of the models for predicting salt uptake in lean considerably decreases.

In another preferred embodiment the method comprises the steps of obtaining the initial percentage by weight of loin and/or fat of said meat product, adding said initial percentage by weight of loin and/or fat to said models of correlation and obtaining an estimated value of the water-holding capacity and/or salt uptake in lean and/or intramuscular fat content of said meat product based on said models of correlation. The importance of this additional parameter is based on the fact that the dielectric properties of the meat product, particularly at frequencies above 100 kHz, depend on the amount of muscle tissue present in the meat product. Therefore, normalizing the quadrature components to the lean tissue mass allows decoupling this effect, improving meat product quality parameter prediction capacity.

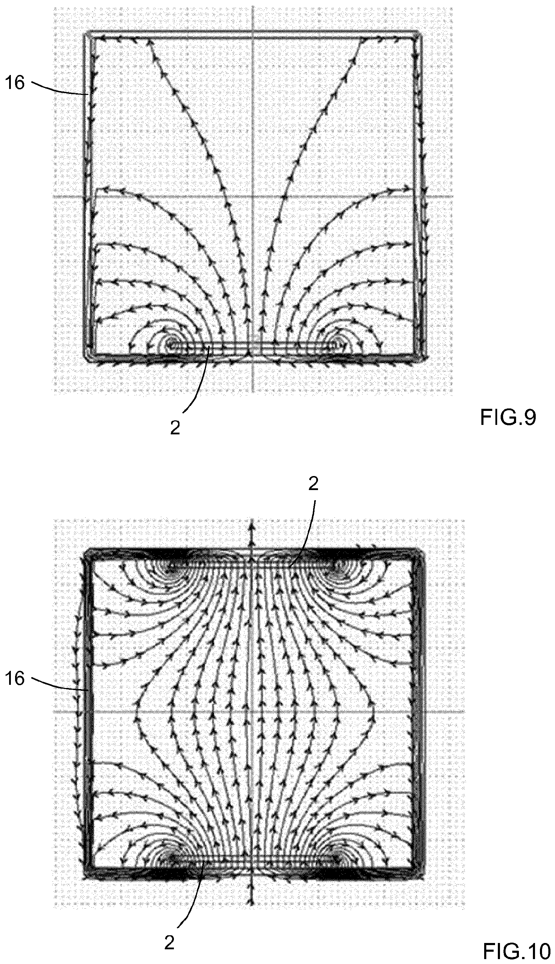

In addition, for the purpose of achieving greater directivity and homogeneity of the primary electromagnetic field, as well as greater directivity in detecting the total electromagnetic field, in a preferred embodiment the method comprises the step of providing first and second transmitter coils and first and second receiver coils, and the primary electromagnetic fields generated by said two transmitter coils have a 0 or 180° phase shift, depending on the winding direction of the wires thereof, and the estimate of the secondary electromagnetic field is made based on adding or subtracting the secondary electromagnetic fields obtained from said two receiver coils. In this configuration, the two transmitter coils are excited with signals having a 180° phase shift, such that the primary field consisting of the superposition of the fields of each of the transmitter coils has the particularity of being strongly confined in the space between the two coils. Similarly, improvement in directivity of the receiving system is achieved by subtracting the signals received in the two receiver coils. To graphically illustrate the effect of confining the primary electromagnetic field, Figure 10 shows a simulation of the distributions of the field lines obtained using a single transmitter coil, and two transmitter coils that have a 180° phase shift.

In a particularly preferred manner, the method according to the invention comprises the steps of generating in said at least one transmitter coil a plurality of primary electromagnetic fields at different excitation frequencies, and determining the in-phase and/or quadrature components of the total electromagnetic fields for each of said excitation frequencies by means of said at least one receiver coil, obtaining a model of correlation comprising said in-phase and/or quadrature components of each of said total electromagnetic fields for each of said excitation frequencies, and obtaining an estimated value of the water-holding capacity and/or salt uptake in lean and/or intramuscular fat content of said meat product based on each of said models of correlation. In a particularly preferred manner, the method comprises implementing said at least one transmitter coil and said at least one receiver coil in the form of tunable coils, which allows obtaining the response of the meat product at multiple excitation frequencies, even in the case of using a single transmitter coil and a single receiver coil. It has additionally been found that excitation frequencies being comprised in a range comprised between 1 kHz to 100 MHz is particularly advantageous for the meat products.

Also for the purpose of calibrating the measurement of the phase and quadrature components, the method according to the invention comprises the steps of obtaining the total electromagnetic field in the absence of a meat product by means of determining in said at least one receiver coil the in-phase and quadrature components of the total electromagnetic field in the absence of said meat product, determining the phase shift between the total electromagnetic field and the primary electromagnetic field in the absence of said meat product, and correcting said phase shift.

Preferably and for the purpose of maximizing the dynamic measurement range of the instrument, the method also additionally comprises the steps of obtaining the total electromagnetic field in the absence of a meat product by means of determining in said at least one receiver coil the in-phase and/or quadrature component of said total electromagnetic field in the absence of said meat product, generating an electric compensation signal with a phase and a quadrature such that they cancel out the in-phase and quadrature components of the total electromagnetic field in said at least one receiver coil due to the primary electromagnetic field in the absence of a meat product. Comparatively, the primary electromagnetic field is much more intense than the secondary electromagnetic field generated in the meat product (usually, several orders of magnitude greater). Accordingly, in the absence of an electric compensation signal, the total electromagnetic field consists of the superposition of a component associated with the secondary electromagnetic field generated in the meat product and an additional component associated with the primary electromagnetic field, which conceals the contribution of interest associated with the secondary field. Therefore, as a result of the electric compensation signal, the signal detected in the receiver coil only corresponds to the signal produced by the meat product, and such signal can be freely amplified, taking advantage of the entire dynamic range of the acquisition system and substantially improving measurement precision.

In an alternative embodiment, the method additionally comprises the steps of obtaining the amplitude of the electric compensation signal needed for cancelling out the total electromagnetic field in the absence of a meat product, normalizing the values of the in-phase and quadrature components of the total field in the presence of a meat product by means of the process of dividing said in-phase and quadrature components by the value of said amplitude of the electric compensation signal, obtaining a model of correlation comprising said normalized in-phase and/or quadrature components of the total electromagnetic field for each of said excitation frequencies, and obtaining an estimated value of the water-holding capacity and/or salt uptake in lean and/or intramuscular fat content of said meat product based on each of said models of correlation. This method of normalization allows compensating for possible system drifts, improving measurement reproducibility and reliability.

Optionally, the method also comprises the steps of providing vision means, determining by means of said vision means at least one morphological parameter of said meat product selected from height, length, width, area and/or volume of said meat product, and adding one or several of said morphological parameters to said model of correlation, and obtaining an estimated value of the water-holding capacity and/or salt uptake in lean and/or intramuscular fat content of said meat product based on said models of correlation. Alternatively or complementarily to the initial weight of the meat product, the use of said morphological parameters allows improving the precision of correlation models, considering the relationship between the geometry of the meat product and the mutual induction coefficient between transmitter coil, meat product and receiver coil. Given a specific configuration, it is possible to establish terms for correcting models of correlation based on system response modeling either by means of analytical techniques and/or empirical methods based on finite element simulation.

Optionally, the method also comprises the steps of providing vision means, determining by means of said vision means at least one color parameter, expressed in the CIELAB color space, in the lean area of said meat product selected from luminance (L*) and/or the red/green level (a*) and/or the blue/yellow level (b*) of said meat product, and adding one or several of said color parameters (L*a*b*) to said model of correlation and obtaining an estimated value of the water-holding capacity of said meat product based on said models of correlation. The inclusion of said color coordinates improves the obtained correlation because during development of the invention, it has been found that this group of variables, particularly luminance (L*), is a good water-holding capacity estimator. The CIELAB color space refers to the color model used to describe all colors that can be perceived by the human eye.

In a particularly preferred manner, the method according to the invention comprises a prior image processing step consisting of removing from the image obtained by said vision means pixels corresponding to connective tissue and intramuscular fat before determining said color parameter.

The invention also proposes an installation of the type indicated above for putting the invention into practice, which installation in its most generic form is characterized in that in addition to the features described at the beginning of the description, it further comprises a magnetic circuit of a material chosen from a ferromagnetic, ferrimagnetic and/or superparamagnetic material, said magnetic circuit being suitable for confining the electromagnetic flux between said at least one transmitter coil and said at least one receiver coil in an inspection volume, said inspection volume being configured such that it allows subjecting said meat product to the effects of said primary electromagnetic field for inducing currents in said meat product, generating a secondary electromagnetic field, and detecting the total electromagnetic field in said at least one receiver coil.

The purpose of this magnetic circuit is to confine the electromagnetic field inside the inspection volume of the meat product such that it directs the magnetic flux from the transmitter coil to the receiver coil in a controlled and homogenous manner. The consequence of doing so is an improvement of an order of magnitude in system sensitivity, and accordingly, an increase in the precision of the installation. At the same time, said magnetic circuit also minimizes the generation of currents induced in other cuts or structural elements that are nearby which could affect measurement reproducibility and/or sensitivity, therefore improving measurement reliability. As a result, relatively large meat products that are close to one another can be inspected with satisfactory results that are representative of the product as a whole in a much more reliable manner than with devices of the state of the art.

Furthermore, in its preferred embodiments the magnetic circuit has an O- or C-shaped configuration, also including an inverted C-shaped configuration.

One of the important objectives of the installation according to the invention is measurement precision. Therefore, throughout the invention it has been found that precision considerably improves preferably when said at least one transmitter coil and said at least one receiver coil are in the center of said installation facing one another, which allows increasing the primary electromagnetic field flux in the central area of the installation and, accordingly, improving precision.

Also, particularly when meat products are bulky and have irregular shapes, such as hams, for example, the configuration of the arrangement of the coils can negatively affect measurement precision. In addition, it is also important that the quality parameters be determined as quickly as possible. Therefore, the installation according to the invention preferably comprises a conveyor belt defining a longitudinal conveying direction, and in which said at least one transmitter coil is provided below said conveyor belt, whereas said at least one receiver coil is provided above said conveyor belt. Alternatively, said at least one transmitter coil and said at least one receiver coil can be interchanged, such that said at least one receiver coil is provided below the conveyor belt and said at least one transmitter coil is provided above said conveyor belt. This arrangement is more favorable because it allows taking advantage of the fact that the distance from the cut to the coils once it is placed on the belt is fixed, thereby preventing possible errors associated with positioning the cut on the belt.

In addition, it has been found that variations in the positioning of the cut on the transverse axis of the belt can negatively affect the estimate of the features of the cut. To minimize this effect, it is particularly important to maximize homogeneity of the primary electromagnetic field within the inspection volume. One way of obtaining said homogeneity consists of using a configuration in which said at least one transmitter coil and said at least one receiver coil have a configuration such that the width thereof is at least 20% greater than the width of the meat product, said width being defined in the plane of conveyance according to the direction perpendicular to the longitudinal forward movement direction of said conveyor belt. This assures that the flux density associated with the primary electromagnetic field is homogenous within a cross section that is larger than that taken up by the meat product, improving measurement precision and reproducibility.

Therefore, a preferred implementation of the system is based on said at least one transmitter coil and said at least one receiver coil being rectangular, the width thereof being greater than the length thereof, and being oriented such that the width thereof is perpendicular to the longitudinal forward movement direction.

The installation according to the invention preferably comprises a plurality of transmitter and receiver coils in the same number, and in which said transmitter coils are simultaneously or sequentially excitable.

The installation preferably comprises the use of said at least one transmitter coil and at least one receiver coil that are tunable, which allows characterizing the response of the meat product at multiple frequencies using a reduced number of transmitter coils and receiver coils.

In an alternative embodiment, the installation according to the invention comprises vision means for determining morphological parameters in said meat product selected from height, length, width, area and/or volume of said meat product.

Finally, in an alternative embodiment the installation according to the invention comprises vision means for determining in said meat product the CIELAB color coordinates thereof such that the water-holding capacity can be determined.

Likewise, the invention also includes other features of detail illustrated in the detailed description of an embodiment of the invention and in the accompanying figures.

Brief description of the drawings

Further advantages and features of the invention will become apparent from the following description, in which, without any limiting character, preferred embodiments of the invention are disclosed, with reference to the accompanying drawings in which:

Figure 1 shows a longitudinally cut schematic side view of an installation for determining quality parameters in meat products according to the invention.
Figure 2 shows a schematic front view of the installation of Figure 1.
Figure 3 shows a schematic front view of a second embodiment of the installation according to the invention.
Figure 4 shows a longitudinally cut schematic side view of a third embodiment of the installation according to the invention.
Figure 5 shows a longitudinally cut schematic side view of a fourth embodiment of the installation according to the invention.
Figure 6 shows the estimated salt concentration values in loin in hams, obtained by means of a partial least squares regression (PLSR) model using 5 main components.
Figure 7 shows the estimated values of drip loss in tenderloins based on a PLSR model using 7 main components.
Figure 8 shows the estimated values of intramuscular fat in hams based on a PSLR model using 5 main components.
Figure 9 shows a simulation of the distribution of the primary electromagnetic field lines obtained from a single transmitter coil.
Figure 10 shows a simulation of the distribution of the primary electromagnetic field lines obtained from two transmitter coils having a 180° phase shift according to the arrangement of coils in Figure 5.

Detailed description of embodiments of the invention

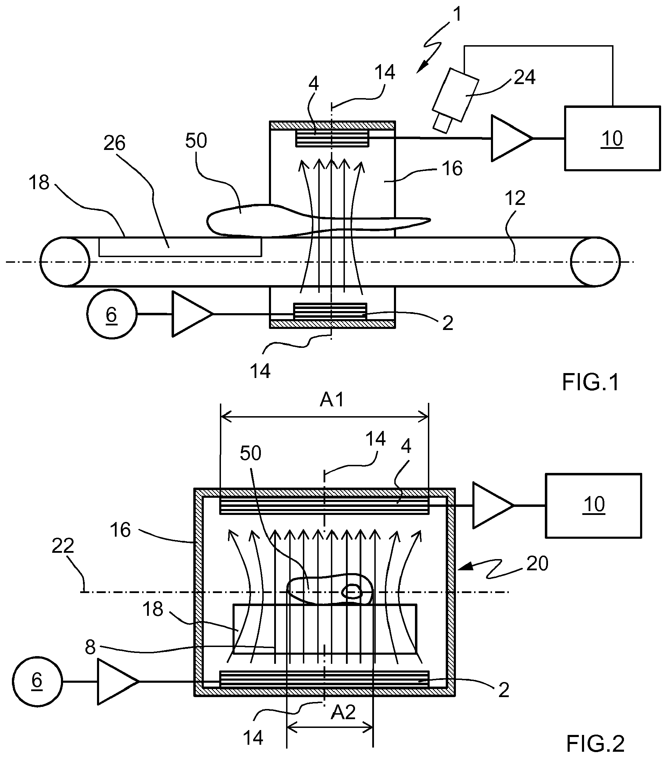

Figure 1 schematically shows an installation 1 for determining quality parameters in meat products according to the invention.

The installation 1 has a longitudinal conveying direction 12 defined based on a conveyor belt 18 on which the meat product 50 rests, said meat product 50 in this embodiment being a ham. At the beginning of the conveyor belt 18, the installation comprises a scale 26 for determining the weight of the meat product 50 passing through the installation 1. Nevertheless, determining the weight in the production line is not essential for the invention because it could be done in another separate installation. It should also be mentioned that the conveyor belt 18 is not essential either because for small-capacity installations, such as installations intended for a laboratory, for example, the method according to the invention could be carried out in an interrupted manner in an installation not conveying the meat product.

In the vertical direction, a transmitter coil 2 is provided below the conveyor belt 18, whereas a receiver coil 4 is provided above it. In this embodiment, both coils 2, 4 are facing one another in the vertical direction of the installation. In addition, it can be seen that the winding axis 14 of the transmitter and receiver coils 2, 4 is transverse to the longitudinal direction 12, and in this case more specifically perpendicular to the plane going through the support surface for supporting the meat product 50 on the conveyor belt 18. The position of the transmitter and receiver coils 2, 4 could alternatively be interchanged, the receiver coil being provided above the conveyor belt 18 and the receiver coil 4 being provided below it.

As schematically indicated in Figures 1 and 2 through the field lines 8, this configuration generates a very homogenous electromagnetic field in the vertical direction, particularly in the central area of the coils 2, 4. Measurement errors due to variation of the cross section of the meat product 50 are therefore considerably reduced.

Figure 2 shows that the installation 1 also has generating means 6 connected to the transmitter coil 2. These generating means 6 will preferably be a multifrequency electric signal generator in order to carry out a frequency sweep, thereby improving characterization of the dielectric properties of the meat product 50 and obtaining an improvement in the prediction capacity of the models of correlation. The coils 2, 4 are also preferably tunable.

On the side of the receiver coil 4, the installation has determination means 10 for determining the in-phase and/or quadrature components of the total electromagnetic field relative to the primary electromagnetic field from the transmitter coil 2.

Finally, Figure 2 shows that the installation 1 comprises a magnetic circuit 16 having a square cross section, with the axis thereof being oriented parallel to the longitudinal direction 12, describing an O shape. The magnetic circuit 16 is of a material selected from a ferromagnetic, ferrimagnetic and superparamagnetic material, or combinations thereof.

As a result of its O-shaped configuration and the permeability characteristics of any of these three materials, the electromagnetic flux between the lower transmitter coil 2 and upper receiver coil 4 is confined in a closed inspection volume 20. The inspection volume 20 is as narrow as possible in the longitudinal direction 12. By way of example, Figure 9 schematically shows the effect of the magnetic circuit 16 with a transmitter coil 2. Said figure clearly shows that the primary electromagnetic field is confined in the inspection volume as a result of the action of a high-permeability ferrimagnetic shield ($\mu$=2000). Accordingly, as a result of the magnetic circuit 16 homogeneity of the electromagnetic field in the event of variations in dimension and/or position of the meat product 50 relative to the transverse axis 22 is improved, and the field is better directed. All this increases sensitivity by at least one order of magnitude and allows inspecting larger and more irregular cuts.

Also in Figure 2, the transmitter and receiver coils 2, 4 are rectangular and wider than the meat product 50, and in a particularly preferred manner at least 20% wider than the width of the meat product 50. They are furthermore arranged such that the width thereof is perpendicular to the longitudinal direction 12.

Finally, Figures 1 to 2 show that the installation 1 comprises vision means 24 in the form of cameras connected to the determination means 10. These cameras determine morphological parameters in the meat product 50, such as height, length, width, area and/or volume. As proposed by the method according to the invention, they can be added to the models of correlation for gaining precision in estimating quality parameters. The vision means 24 can be used in a complementary manner for determining color parameters of the meat product 50. In a particularly preferred manner, the color parameter is expressed in the CIELAB color space. This color parameter, in the lean area of the meat product 50 is selected from luminance L* and/or red/green level a* and/or blue/yellow level b* of the meat product 50. These color parameters L*, a*, b* can then be added to the model of correlation. An estimated value of the water-holding capacity of the meat product 50 can be obtained from this new, more precise model.

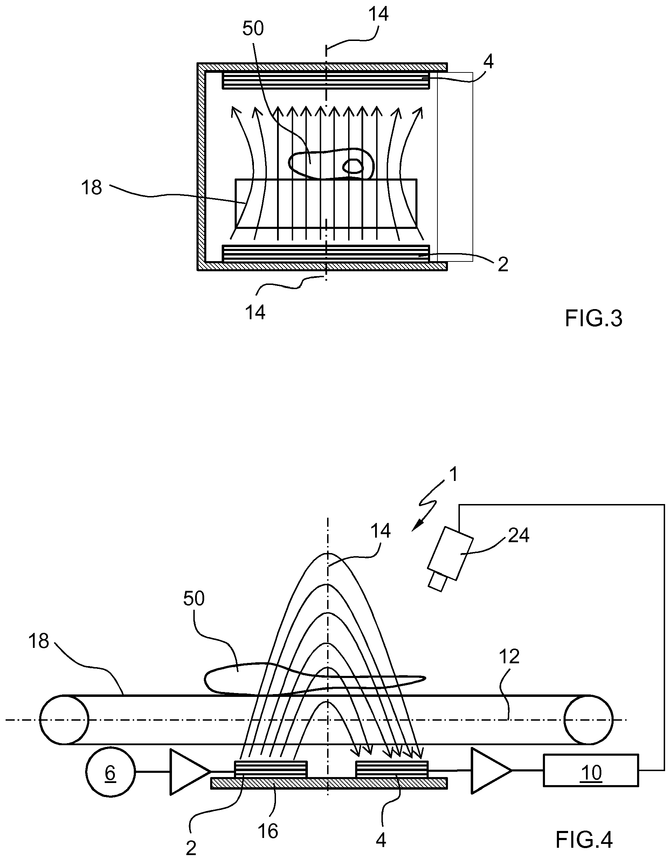

Figure 3 shows an alternative embodiment in which the magnetic circuit 16 is C-shaped, with the transmitter and receiver coils 2, 4 facing one another on both sides of the conveyor belt 18 in the vertical direction. The C-shaped configuration can obviously be both a conventional C shape and a C shape (left-inverted C shape), without this changing the scope of protection of the invention.

Figure 4 shows a third embodiment of the invention which is substantially similar to the embodiment in Figures 1 and 2. Nevertheless, in this case, the transmitter and receiver coils 2, 4 are both located below the conveyor belt 18 and connected to one another by means of the magnetic circuit 16. This offers flexibility to the installation 1 because as a result of this configuration, meat products having a larger volume can be analyzed without the limitation of them going through the magnetic circuit 16.

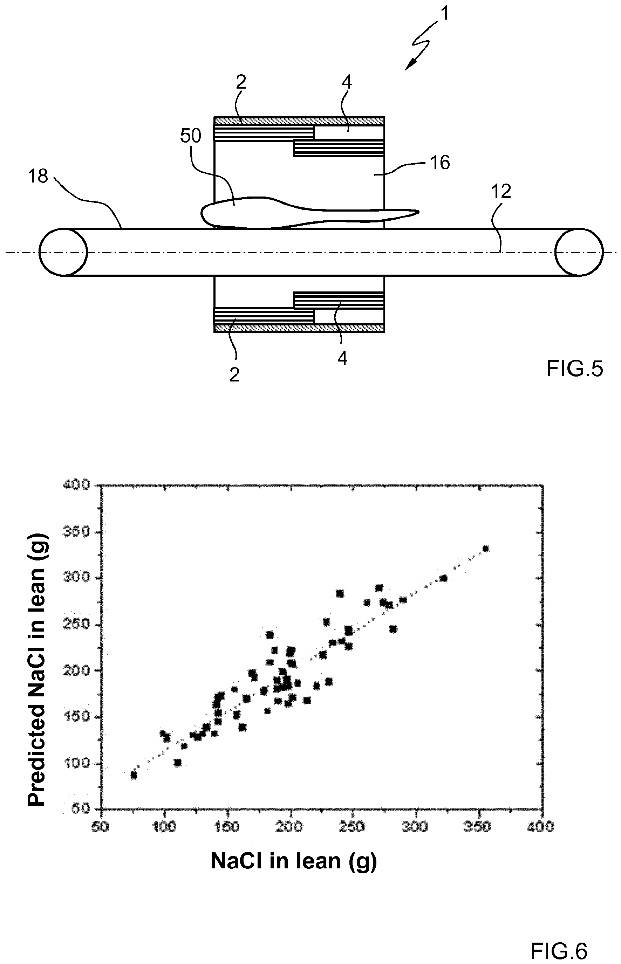

Figure 5 shows a fourth embodiment of the installation 1 of the invention in which two transmitter coils 2 and two receiver coils 4 are provided. One transmitter coil 2 and one receiver coil 4 are located in the lower part of the conveyor belt 18, whereas the other two coils 2, 4 are located in the upper part. Furthermore, each receiver coil 4 can be partially overlapped with its corresponding transmitter coil 2, which allows partially compensating for the field induced in the receiver coils 4 as a result of the primary field. This configuration allows cancelling the radial components of the electromagnetic field when the electric signals exciting the transmitter coils have a 180° phase shift, obtaining a very homogenous electromagnetic field in the axial direction of the coils, and confining it in the longitudinal direction. This effect can be more clearly seen in Figure 10, where the greater homogeneity of the electromagnetic field can be seen.

Figure 6 shows the results obtained with the method according to the invention in a first embodiment for determining the salt, NaCl, uptake in lean. The salt uptake in lean in sixty Parma hams was analyzed in this test.

The hams were subjected to an individual salting process for a period of 15 days. After salting with common salt, the hams were brushed and washed to remove salt residue on the surface. The salt, NaCl, uptake in lean was measured by means of analytical determination following standard protocols.

The initial weight W of the ham before salting was determined in a prior phase. Then weight loss WL after salting was determined as a difference between the initial weight W of the ham and the final weight after salting.

Next the hams were subjected in the installation 1 to the effects of the primary electromagnetic field generated by the transmitter coil 2. The electromagnetic response was obtained in the receiver coil 4 by determining the in-phase components I and quadrature components Q of the total electromagnetic field at two different frequencies: 1900 kHz and 725 kHz. Morphological parameters of the cuts (height, width, and maximum length) were also obtained by means of a viewing system.

The obtained results show that the in-phase and quadrature components of the total electromagnetic field can be used for establishing models of correlation for predicting salt uptake in lean. The inclusion of additional parameters also allows reducing the prediction error of models of correlation.

Remarkably, it has been found through experiments that it is possible to obtain a good correlation even by using only the quadrature component of the total electromagnetic field at 1.9 MHz and weight loss after salting. By way of illustration, the prediction equation obtained in this case based on a multilinear equation is provided below.

$$NaCl[g]=-35.5+31.17 \cdot WL[Kg]+2.06 \cdot Q[V]/W[kg]$$

Figure 6 shows the obtained curve from which salt uptake in lean can be determined using the preceding equation.

The use of advanced statistical methods such as partial least squares regression (PLSR) allows establishing more precise models of correlation, including the number of variables considered. Based on the analysis of the experimental results, it has been established that a model of correlation with 5 main components allows reducing the prediction error of salt uptake in lean to limits that are comparable to those of the reference method, with corrected coefficients of determination of Adj. $R^2 = 0.86$ and with a RMSE prediction error = 20 g.

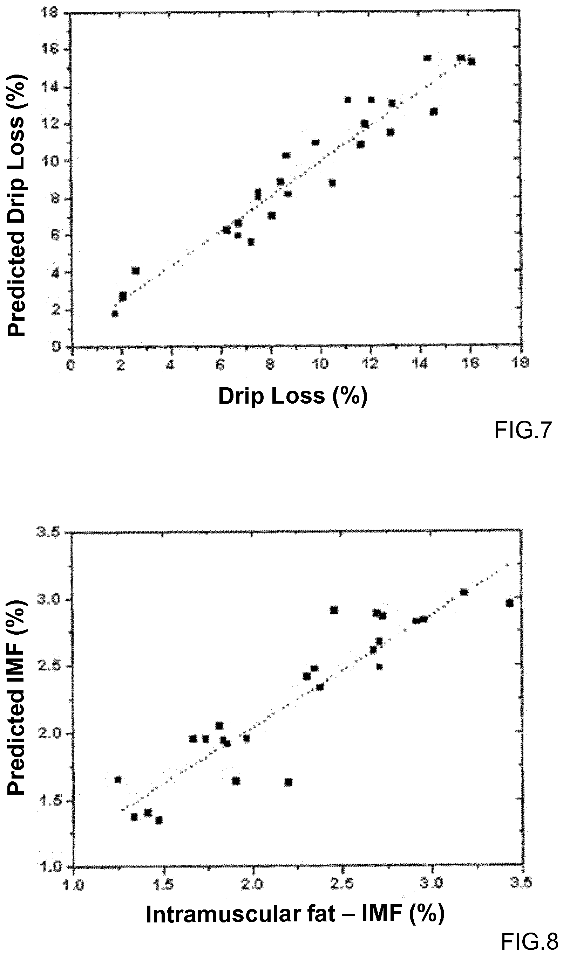

Figure 7 shows a second embodiment of the method according to the invention in the case of determining water-holding capacity.

In this second example, a total of 26 white pork tenderloin samples were analyzed. Water-holding capacity was determined by evaluating drip loss in 24 hours, following the normalized Honikel's Reference Bag Drip Method, considered to be the standard method in the industry for determining water-holding capacity.

The following parameters are determined in each case:

- Drip loss: DL%
- Weight of the cut: W
- CIELAB color coordinates of the lean surface (L* a* b*)
- Electric phase and quadrature signals at 10 different frequencies: { Ij Qj}

The excitation frequencies used were: 25 kHz, 40 kHz, 50 kHz, 85 kHz, 100 kHz, 200 kHz, 300 kHz, 400 kHz, 600 kHz and 850 kHz.

By means of models of linear correlation it can be seen that the induction spectrum is correlated to the target variable (DL%), and particularly the relationships between quadrature signals at different frequencies. For example, a simple linear model using only the weight and quotient Q100/Q40 provides correlations of Adj. $R^2 = 0.76$ and RMSE=2, Adj. $R^2$ being the adjusted correlation coefficient and RMSE being the root mean square error.

A multilinear model including the quotients Q100/40, Q300/85 and Q600/400 was used to improve the estimate of the water-holding capacity. Said model provides correlations of Adj. $R^2 = 0.82$ and RMSE=1.7.

The partial least squares (PLS) regression model, which includes all the experimentally determined variables ((Qi/Qj) [26x45], L*, a*, b*, w), is used to obtain an optimal model of correlation. It is established that the optimal number of variables in the model is 7 (number of main components).

The correlation achieved with the PLS model is Adj. $R^2 = 0.94$, with an RMSE calibration error = 1%.

Figure 8 shows a third embodiment of the method according to the invention in the case of determining intramuscular fat.

In this third example, a total of 30 hams with a percentage of intramuscular fat between 1 and 10% were analyzed. The percentage of intramuscular fat was determined by means of the Soxhlet chemical extraction method, considered to be the standard method in the industry for determining fat content.

The following parameters were determined in each case:

- % of intramuscular fat (IMF): IMF%
- Weight of the cut: W
- Electric phase and quadrature signals at 10 different frequencies: {Ij Qj}

The excitation frequencies used were: 25 kHz, 40 kHz, 50 kHz, 85 kHz, 100 kHz, 200 kHz, 300 kHz, 400 kHz, 600 kHz and 850 kHz.

By means of models of linear correlation it can be seen that the induction spectrum is correlated to the target variable (IMF%), and particularly the relationships between quadrature signals at different frequencies. For example, a simple linear model using only the weight and quotient $Q_{850}/Q_{40}$ provides correlations of Adj. $R^2 = 0.76$ and RMSE=0.3

$$IMF\ [\%] = 5.318 - (4.100 \cdot Q_{850}/Q_{40})/W$$

As in previous cases, the inclusion of all the variables considered in the model considerably improves the correlation. Particularly, a model based on partial least squares regression using 5 main components allows improving the adjusted coefficient of determination (Adj. $R^2 = 0.85$), reducing the prediction error to RMSE = 0.2. Figure 8 shows the results obtained based on this model.

**Claims**

1. An installation for determining quality parameters in meat products comprising

   [a] at least one transmitter coil (2),
   [b] at least one receiver coil (4),
   [c] generating means (6) for generating a primary time-variable electromagnetic field in said at least one transmitter coil (2),
   [d] determination means (10) for determining in said at least one receiver coil (4) the in-phase and/or quadrature components of the total electromagnetic field,

   **characterized in that** it further comprises a magnetic circuit (16) of a material chosen from a ferromagnetic, ferrimagnetic and/or superparamagnetic material, said magnetic circuit (16) being suitable for confining the electromagnetic flux between said at least one transmitter coil (2) and said at least one receiver coil (4) in an inspection volume, said inspection volume (20) being configured such that it allows subjecting said meat product (50) to the effects of said primary electromagnetic field for inducing currents in said meat product (50), generating a secondary electromagnetic field, and detecting the total electromagnetic field in said at least one receiver coil (4).

2. The installation for determining quality parameters in meat products according to claim 1, **characterized in that** said magnetic circuit (16) has an O- or C-shaped configuration.

3. The installation for determining quality parameters in meat products according to claim 1 or 2, **characterized in that** said at least one transmitter coil and said at least one receiver coil (2, 4) are in the center of said installation facing one another.

4. The installation for determining quality parameters in meat products according to any of claims 1 to 3, **characterized in that** it comprises a conveyor belt (18) defining a longitudinal conveying direction, and **in that** said at least one transmitter coil (2) is provided below said conveyor belt (18), whereas said at least one receiver coil (4) is provided above said conveyor belt (18).

5. The installation for determining quality parameters in meat products according to claim 4, **characterized in that** said at least one transmitter coil (2) and said at least one receiver coil (4) are interchanged, such that said at least one receiver coil (4) is provided below the conveyor belt (18) and said at least one transmitter coil (2) is provided above said conveyor belt (18).

6. The installation for determining quality parameters in meat products according to any of claims 1 to 5, **characterized in that** said at least one transmitter coil and said at least one receiver coil (2, 4) have a configuration such that the width thereof is at least 20% greater than the width of the meat product (50), said width being defined in the plane of conveyance according to the direction perpendicular to the longitudinal forward movement direction of said conveyor belt (18).

7.   The installation for determining quality parameters in meat products according to claim 6, **characterized in that** said at least one transmitter coil and said at least one receiver coil (2, 4) are rectangular, the width thereof being greater than the length thereof, and being oriented such that the width thereof is perpendicular to said longitudinal direction.

8.   The installation for determining quality parameters in meat products according to any of claims 1 to 7, **characterized in that** it comprises a plurality of transmitter and receiver coils (2, 4), and **in that** said transmitter coils (2) are simultaneously or sequentially excitable.

9.   The installation for determining quality parameters in meat products according to any of claims 1 to 8, **characterized in that** it comprises vision means (24) for determining morphological parameters in said meat product (50) selected from height, length, width, area and/or volume of said meat product (50).

10.   The installation for determining quality parameters in meat products according to any of claims 1 to 9, **characterized in that** it comprises vision means (24) for determining in said meat product (50) the CIELAB color coordinates thereof such that the prediction error for the water-holding capacity can be improved.

FIG.1

FIG.2

FIG.3

FIG.4

FIG.5

FIG.6

FIG.7

FIG.8

16

2

FIG.9

2

16

2

FIG.10

Europäisches
Patentamt

European
Patent Office

Office européen
des brevets

## EUROPEAN SEARCH REPORT

Application Number

EP 18 18 4665

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 0 375 366 A2 (LOMA GROUP LTD [GB]) 27 June 1990 (1990-06-27) * column 7, line 4 - line 13 * * column 11, line 14 - column 12, line 43 * * figure 1 * | 1-10 | INV. G01N27/02 G01N33/12 |
| Y | GB 1 436 900 A (HEYTOW S) 26 May 1976 (1976-05-26) * page 2, line 71 - line 104 * * figure 7 * | 1-10 | |
| A | US 2012/179394 A1 (KITTEL CLIVE FRANCIS [GB] ET AL) 12 July 2012 (2012-07-12) * paragraph [0045] * * figures 4-6 * | 1-10 | |
| A | FR 2 843 636 A1 (FRANCK MICHEL [FR]) 20 February 2004 (2004-02-20) * the whole document * | 1-10 | |
| | | | TECHNICAL FIELDS SEARCHED (IPC) |
| | | | G01N G01V |

The present search report has been drawn up for all claims

| Place of search | Date of completion of the search | Examiner |
|---|---|---|
| The Hague | 6 November 2018 | Baranski, Jörg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 425 380 A1**

## ANNEX TO THE EUROPEAN SEARCH REPORT
## ON EUROPEAN PATENT APPLICATION NO.

EP 18 18 4665

This annex lists the patent family members relating to the patent documents cited in the above-mentioned European search report.
The members are as contained in the European Patent Office EDP file on
The European Patent Office is in no way liable for these particulars which are merely given for the purpose of information.

06-11-2018

| Patent document cited in search report | | Publication date | Patent family member(s) | | Publication date |
|---|---|---|---|---|---|
| EP 0375366 | A2 | 27-06-1990 | CA | 2005941 A1 | 20-06-1990 |
| | | | DE | 68918825 D1 | 17-11-1994 |
| | | | DE | 68918825 T2 | 09-02-1995 |
| | | | EP | 0375366 A2 | 27-06-1990 |
| | | | ES | 2060795 T3 | 01-12-1994 |
| | | | JP | H02269955 A | 05-11-1990 |
| | | | US | 5189366 A | 23-02-1993 |
| GB 1436900 | A | 26-05-1976 | AU | 5620573 A | 28-11-1974 |
| | | | CA | 1080821 A | 01-07-1980 |
| | | | DE | 2326797 A1 | 06-12-1973 |
| | | | ES | 415206 A1 | 16-05-1976 |
| | | | FR | 2189756 A1 | 25-01-1974 |
| | | | GB | 1436900 A | 26-05-1976 |
| | | | IL | 42338 A | 15-06-1978 |
| | | | IT | 990589 B | 10-07-1975 |
| | | | JP | S4963462 A | 19-06-1974 |
| US 2012179394 | A1 | 12-07-2012 | GB | 2423366 A | 23-08-2006 |
| | | | GB | 2462212 A | 03-02-2010 |
| | | | US | 2012179394 A1 | 12-07-2012 |
| | | | WO | 2006087510 A1 | 24-08-2006 |
| FR 2843636 | A1 | 20-02-2004 | AU | 2003274265 A1 | 03-03-2004 |
| | | | BR | 0306131 A | 19-10-2004 |
| | | | EP | 1530718 A2 | 18-05-2005 |
| | | | FR | 2843636 A1 | 20-02-2004 |
| | | | WO | 2004017067 A2 | 26-02-2004 |

For more details about this annex : see Official Journal of the European Patent Office, No. 12/82

EPO FORM P0459

18